(19) Europäisches Patentamt
European Patent Office
Office européen des brevets

(11) Veröffentlichungsnummer: **0 561 421 A1**

(12) **EUROPÄISCHE PATENTANMELDUNG**

(21) Anmeldenummer: **93104574.4**

(22) Anmeldetag: **19.03.93**

(51) Int. Cl.[5]: **C07D 213/78**, C07D 241/24

(30) Priorität: **20.03.92 CH 899/92**

(43) Veröffentlichungstag der Anmeldung:
**22.09.93 Patentblatt 93/38**

(84) Benannte Vertragsstaaten:
**AT BE CH DE DK ES FR GB GR IE IT LI NL PT SE**

(71) Anmelder: **LONZA AG**
**Gampel/Wallis(CH)**

(72) Erfinder: **Roduit, Jean-Paul, Dr.**
**Bois-de-Finges 4**
**Sierre (Kanton Wallis)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-80803 München (DE)**

(54) **Verfahren zur Herstellung von Carbonsäurechloriden aromatischer Stickstoff-Heterocyclen.**

(57) Beschrieben wird ein neues Verfahren zur Herstellung von Carbonsäurechloriden aromatischer Stickstoff-Heterocyclen aus der Reihe der Verbindungen der allgemeinen Formel

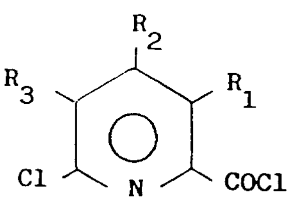

I

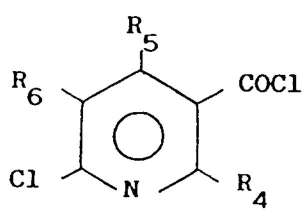

II

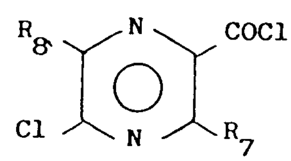

III

durch Chlorierung von Hydroxycarbonsäuren aromatischer Stickstoff-Heterocyclen aus der Reihe der Verbindungen der allgemeinen Formeln

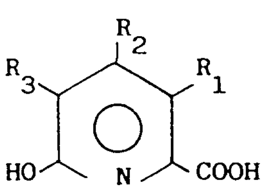

IV

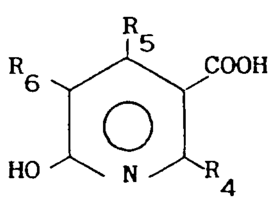

V

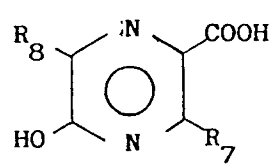

VI

Die Chlorierung wird mit einer Lösung von Phosphorpentachlorid in Phosphoroxychlorid durchgeführt, wobei das

EP 0 561 421 A1

überschüssige Phosphorpentachlorid entweder mit einer $C_1$-$C_4$-Carbonsäure oder mit einem Silan der allgemeinen Formel

$(CH_3)_3$-Si-$OR_9$ VII

in Phosphoroxychlorid überführt wird und anschliessend das Produkt gemäss den Formeln I bis III isoliert wird.

Die Erfindung betrifft ein neues Verfahren zur Herstellung von Carbonsäurechloriden aromatischer Stickstoff-Heterocyclen aus der Reihe der Verbindungen der allgemeinen Formeln

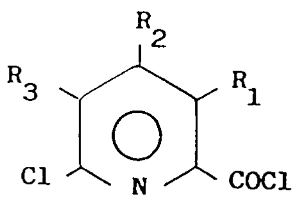

I

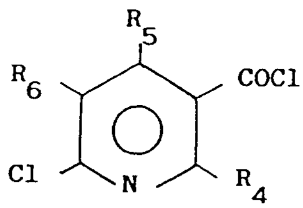

II

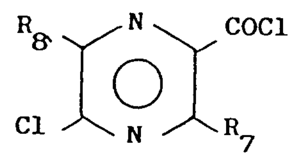

III

durch Chlorierung von Hydroxycarbonsäuren aromatischer Stickstoff-Heterocyclen aus der Reihe der Verbindungen der allgemeinen Formeln

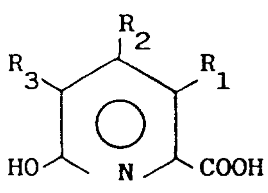

IV

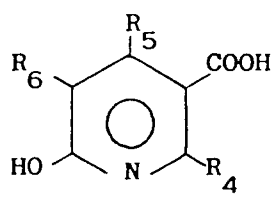

V

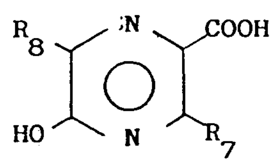

VI

Diese Carbonsäurechloride aromatischer Stickstoff-Heterocyclen, wie beispielsweise 6-Chlorpicolinsäurechlorid sind wichtige Zwischenprodukte zur Herstellung von Herbiziden (EP-A 447 004).

Die direkte Umsetzung von 6-Hydroxypicolinsäure zu 6-Chlorpicolinsäurechlorid ist in der Literatur noch nicht beschrieben. Bekannt ist lediglich die Chlorierung von 6-Hydroxypicolinsäure zu 6-Chlorpicolinsäure mit Phosphorpentachlorid in Phosphoroxychlorid (Ber. D. Chem. ges., 1912, 45, S. 2461). Erst durch eine weitere Chlorierung von 6-Chlorpicolinsäure mit beispielsweise Thionylchlorid kann dann 6-Chlorpicolinsäurechlorid hergestellt werden.

Ein Nachteil dieses Verfahrens besteht darin, dass das Produkt unter diesen Bedingungen nur über eine 2-stufige Synthese hergestellt werden kann und, dass bei der Aufarbeitung der 6-Chlorpicolinsäure als Zwischenprodukt in der ersten Stufe grosse Mengen an Phosphorsäure als Abfallprodukt anfallen.

Des weiteren ist die Herstellung von 6-Chlorpicolinsäurechlorid, ausgehend von 6-Chlor-2-methylpyridin, bekannt (EP-A 032 516).

Ein grosser Nachteil dieses Verfahrens besteht darin, dass man das Edukt 6-Chlor-2-methylpyridin zunächst in einer ersten Stufe mit Kaliumpermanganat zur 6-Chlorpicolinsäure oxidieren muss, die dann mit Thionylchlorid in der zweiten Stufe in das 6-Chlorpicolinsäurechlorid überführt wird, wobei das Produkt in mässiger Ausbeute erhalten wird.

Aufgabe der vorliegenden Erfindung war ein einfaches und wirtschaftliches Verfahren zur Herstellung von Carbonsäurechloriden aromatischer Stickstoff-Heterocyclen zur Verfügung zu stellen, wobei die Produkte in sehr guten Ausbeuten isoliert werden können. Es sollen auch keine größeren Mengen an Phosphorsäure als Abfallprodukt anfallen.

Erfindungsgemäss wird diese Aufgabe gemäss Patentanspruch 1 gelöst.

Das Verfahren zur Herstellung von Carbonsäurechloriden aromatischer Stickstoff-Heterocyclen aus der Reihe der Verbindungen der allgemeinen Formeln

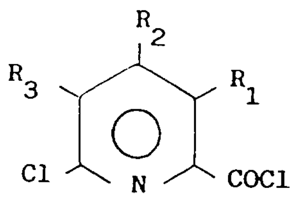 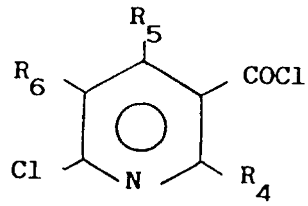 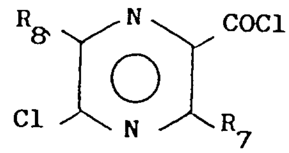

I II III

worin $R_1$, $R_2$, $R_3$, $R_4$, $R_5$, $R_6$, $R_7$ und $R_8$ gleich oder verschieden sind und jeweils ein Wasserstoff- oder Halogenatom, vorzugsweise Cl, bedeuten, durch Chlorierung von Hydroxycarbonsäuren aromatischer Stickstoff-Heterocyclen aus der Reihe der Verbindungen der allgemeinen Formel

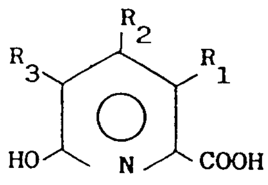 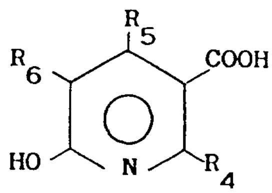 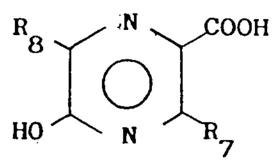

IV V VI

worin $R_1$ bis $R_8$ die genannte Bedeutung haben, wird erfindungsgemäss derart durchgeführt, dass man die Chlorierung mit einer Lösung von Phosphorpentachlorid in Phosphoroxychlorid durchführt, dann das überschüssige Phosphorpentachlorid entweder mit einer $C_1$-$C_4$-Carbonsäure oder mit einem Silan der allgemeinen Formel

$(CH_3)_3$-Si-$OR_9$ VII

worin $R_9$ eine $C_1$-$C_4$-Alkyl oder eine Tri($C_1$-$C_4$)alkylsilylgruppe bedeutet, in Phosphoroxychlorid überführt und anschliessend das Produkt gemäss den Formeln I bis III isoliert.

Ein besonderer Vorteil dieses Verfahrens besteht darin, dass die Carbonsäurechloride aromatischer Stickstoff-Heterocyclen durch den Zusatz von leicht zugänglichen Reagenzien, wie beispielsweise Ameisensäure, isoliert werden können.

Die Edukte, die aromatischen Hydroxycarbonsäuren der Stickstoff-Heterocyclen, können auf einfache Weise mikrobiologisch hergestellt werden, beispielsweise gemäss dem schweizerischen Patentgesuch Nummer 3572/91. (Europäische Patentanmeldung 92 120 757.7)

Als Edukte dienen Hydroxycarbonsäuren aromatischer Stickstoff-Heterocyclen aus der Reihe der Verbindungen der allgemeinen Formeln IV bis VI.

Vorzugsweise werden als Hydroxycarbonsäuren aromatischer Stickstoff-Heterocyclen der allgemeinen Formel IV, 6-Hydroxypicolinsäure, worin $R_1$, $R_2$ und $R_3$ jeweils ein Wasserstoffatom bedeuten oder 6-Hydroxy-3,5-dichlorpicolinsäure, worin $R_2$ ein Wasserstoffatom und $R_1$ und $R_3$ ein Chloratom bedeuten, angewendet. Als aromatische Hydroxycarbonsäure aromatischer Stickstoff-Heterocyclen der allgemeinen Formel VI wird vorzugsweise 5-Hydroxypyrazincarbonsäure, worin $R_7$ und $R_8$ ein Wasserstoffatom bedeuten, angewendet.

Die Chlorierung der Edukte mit Phosphorpentachlorid in Phosphoroxychlorid erfolgt üblicherweise mit einem Überschuss an Phosphorpentachlorid bezüglich der eingesetzten Menge an Edukt.

Zweckmässig wird Phosphorpentachlorid in einer Menge von 1 bis 3 mol, vorzugsweise von 2 bis 2,5 mol, pro mol Edukt (Hydroxycarbonsäure aromatischer Stickstoff-Heterocyclen) eingesetzt.

Zweckmässig wird die Chlorierung bei einer Temperatur von 20 bis 110°C, vorzugsweise von 60 bis 110°C durchgeführt.

Nach einer üblichen Chlorierungsdauer von 2 bis 15 h, zweckmässig bis kein HCl-Gas mehr gebildet wird, wird dann erfindungsgemäss das überschüssige Phosphorpentachlorid entweder mit einer $C_1$-$C_4$-Carbonsäure oder mit einem Silan der allgemeinen Formel VII in Phosphoroxychlorid überführt.

Als $C_1$-$C_4$-Carbonsäuren können beispielsweise Ameisensäure, Essigsäure oder Propionsäure angewendet werden. Vorzugsweise wird als $C_1$-$C_4$-Carbonsäure Ameisensäure angewendet, wobei Kohlenmonoxid und HCl gebildet wird.

Als Silane der allgemeinen Formel

$(CH_3)_3$-Si-$OR_9$ VII

werden zweckmässig Methoxytrimethylsilan, worin $R_9$ eine Methylgruppe bedeutet oder Hexamethyldisiloxan, worin $R_9$ eine Trimethylsilylgruppe bedeutet, angewendet.

Zweckmässig wird die $C_1$-$C_4$-Carbonsäure bzw. das Silan gemäss Formel VII in einer Menge von 0,1 bis 2 mol, vorzugsweise von 1 bis 1,5 mol pro mol Edukt eingesetzt.

Die Zugabe der $C_1$-$C_4$-Carbonsäure oder des Silans der Formel VII erfolgt zweckmässig bei einer Temperatur von 0 bis 60 ° C, vorzugsweise von 20 bis 40 ° C.

Während oder nach einer üblichen Zugabezeit von 15 bis 60 min, kann das dabei gebildete Phosphoroxychlorid destillativ entfernt werden.

Vorzugsweise wird Phosphoroxychlorid destillativ entfernt und gegebenenfalls in die Reaktionslösung rezirkuliert. Dabei wird reines Phosphoroxychlorid, welches wieder für die Reaktion eingesetzt werden kann, im Überschuss produziert.

Das bisher noch nicht beschriebene, 3,5,6-Trichlorpicolinsäurechlorid als Verbindung der allgemeinen Formel I, worin $R_1$ und $R_3$ jeweils ein Chloratom und $R_2$ ein Wasserstoffatom bedeuten, ist ebenfalls Gegenstand der Erfindung.

Beispiel 1:

6-Chlorpicolinsäurechlorid

Zu einer Suspension von 6-Hydroxypicolinsäure (71,9 mmol; 10 g) in Phosphoroxychlorid (165,4 mmol; 25,3 g) wurden 49,9 g (239,5 mmol) Phosphorpentachlorid bei Raumtemperatur hinzugegeben. Es wurde eine exotherme Reaktion, wobei sich HCl-Gas bildete, beobachtet.
Nach Beendigung dieser exothermen Reaktion wurde die Mischung innerhalb 1,5 h auf 90 ° C erwärmt. Anschliessend wurde diese Temperatur 12 h lang gehalten. Die klare Lösung wurde auf 30 ° C abgekühlt und dann wurde Ameisensäure (93,5 mmol; 4,3 g) zugetropft, wobei HCl- und CO-Gas gebildet wurde.
Nach dem Abdestillieren von Phosphoroxychlorid wurde das 6-Chlorpicolinsäurechlorid bei 90 ° C bei einem Druck von 0,026 mbar destilliert. Es wurden 12 g 6-Chlorpicolinsäurechlorid (68,2 mmol) als weisser Feststoff entsprechend einer Ausbeute von 94,8% erhalten.
Schmp.: 73,1 - 75,8 ° C
$^1$H-NMR: ($CDCl_3$) (360 MHz) $\delta$ 7,66 (d, 9,6 Hz); 7,90 (t, 9,6 Hz); 8,07 (d, 9,6 Hz).

Beispiel 2:

In analoger Weise zu Beispiel 1 wurden 100 g 6-Hydroxypicolinsäure zu 6-Chlorpicolinsäurechlorid chloriert, welches in einer Ausbeute von 97,3% erhalten.

Beispiel 3:

3,5 6-Trichlorpicolinsäurechlorid

Entsprechend zu Beispiel 1 wurden 10 g (48 mmol) 3,5-Dichlor-6-hydroxypicolinsäure mit Phosphorpentachlorid (158,6 mmol; 33,1 g) in Phosphoroxychlorid (171 mmol; 26,3 g) bei einer Temperatur von 110 ° C chloriert. Während der Reaktion wurden durch den Zusatz von Ameisensäure, wie in Beispiel 1, 13 g Phosphoroxychlorid bereits abdestilliert. Nach der Destillation bei 110 ° C und 0,065 mbar wurden 7,8 g 3,5,6-Trichlorpicolinsäurechlorid entsprechend einer Ausbeute von 66% erhalten.
Schmp.: 49 - 52 ° C
$^1$H-NMR: ($CDCl_3$, 360 MHz) 8,00 ppm; (s).

Beispiel 4:

5-Chlorpyrazincarbonsäurechlorid

Entsprechend zu Beispiel 1 wurde 5-Hydroxypyrazin-2-carbonsäure (57,1 mmol; 8 g) mit Phosphorpentachlorid (188,4 mmol; 39,3 g) in Phosphoroxychlorid (131,3 mmol; 20,15 g) bei einer Temperatur von 85 °C zu 5-Chlorpyrazin-2-carbonsäurechlorid umgesetzt. Nach Zusatz von Ameisensäure, wie in Beispiel 1, und Destillation bei 85 °C, 1,3 mbar wurden 6,7 g 5-Chlorpyrazin2-carbonsäurechlorid in Form von weissen Kristallen entsprechend einer Ausbeute von 63% erhalten.
Schmp.: >45 °C
$^{1}$H-NMR: ($CDCl_3$, 360 MHz) δ 8,78 (s); 9,10 (s).

Beispiel 5:

6-Chlorpicolinsäurechlorid

a) Entsprechend zu Beispiel 1 wurde 6-Hydroxypicolinsäure mit Phosphorpentachlorid in Phosphoroxychlorid chloriert. Nach Beendigung der Reaktion, wurden bei 50 °C 10,5 g (100 mmol) Methoxytrimethylsilan hinzugefügt, wobei die Bildung von Chlormethan beobachtet wurde. Nach dem Abdestillieren von Phosphoroxychlorid wurden 12,05 g Produkt entsprechend einer Ausbeute von 95% erhalten.
b) Entsprechend zu Beispiel 5a) wurde 6-Chlorpicolinsäurechlorid mit Hexamethyldisiloxan (16,2 g) hergestellt, wobei 12,05 g Produkt entsprechend einer Ausbeute von 95% erhalten wurden.

**Patentansprüche**

**1.** Verfahren zur Herstellung von Carbonsäurechloriden aromatischer Stickstoff-Heterocyclen aus der Reihe der Verbindungen der allgemeinen Formeln

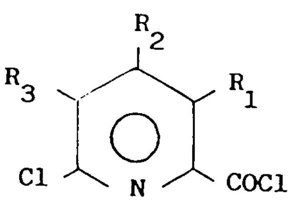
I

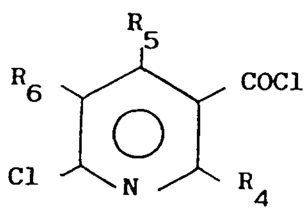
II

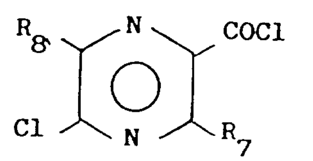
III

worin $R_1$, $R_2$, $R_3$, $R_4$, $R_5$, $R_6$, $R_7$ und $R_8$ gleich oder verschieden sind und jeweils ein Wasserstoff- oder Halogenatom bedeuten durch Chlorierung von Hydroxycarbonsäuren aromatischer Stickstoff-Heterocyclen aus der Reihe der Verbindungen der allgemeinen Formeln

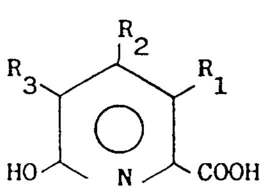
IV

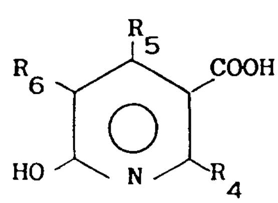
V

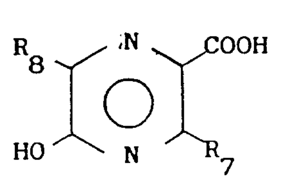
VI

worin $R_1$ bis $R_8$ die genannte Bedeutung haben, dadurch gekennzeichnet, dass man die Chlorierung mit einer Lösung von Phosphorpentachlorid in Phosphoroxychlorid durchführt, dann das überschüssige Phosphorpentachlorid entweder mit einer $C_1$-$C_4$-Carbonsäure oder mit einem Silan der allgemeinen Formel

$(CH_3)_3$-Si-$OR_9$ VII

worin $R_9$ eine $C_1$-$C_4$-Alkyl- oder eine Tri($C_1$-$C_4$)alkylsilylgruppe bedeutet, in Phosphoroxychlorid überführt und anschliessend das Produkt gemäss den Formeln I bis III isoliert.

**2.** Verfahren nach Patentanspruch 1, dadurch gekennzeichnet, dass man als $C_1$-$C_4$-Carbonsäure Ameisensäure verwendet.

**3.** Verfahren nach Patentanspruch 1, dadurch gekennzeichnet, dass man als Silan der allgemeinen Formel VII Methoxytrimethylsilan, worin $R_9$ eine Methylgruppe bedeutet oder Hexamethyldisiloxan, worin $R_9$ eine Trimethylsilylgruppe bedeutet, anwendet.

**4.** Verfahren nach mindestens einem der Patentansprüche 1 bis 3, dadurch gekennzeichnet, dass man als Hydroxycarbonsäure aromatischer Stickstoff-Heterocyclen der allgemeinen Formel IV, 6-Hydroxypicolinsäure, worin $R_1$, $R_2$ und $R_3$ ein Wasserstoffatom bedeuten, 6-Hydroxy-3,5-dichlorpicolinsäure, worin $R_2$ ein Wasserstoffatom, $R_1$ und $R_3$ ein Chloratom bedeuten, oder als Hydroxycarbonsäure aromatischer Stickstoff-Heterocyclen der allgemeinen Formel VI 5-Hydroxypyrazincarbonsäure, worin $R_7$ und $R_8$ ein Wasserstoffatom bedeuten, anwendet.

**5.** Verfahren nach mindestens einem der Patentansprüche 1 bis 4, dadurch gekennzeichnet, dass man die Chlorierung bei einer Temperatur von 20 bis 110 °C durchführt.

**6.** Verfahren nach mindestens einem der Patentansprüche 1 bis 5, dadurch gekennzeichnet, dass man die Zugabe der $C_1$-$C_4$-Carbonsäure oder des Silans der allgemeinen Formel VII bei einer Temperatur von 0 bis 60 °C durchführt.

**7.** Verfahren nach mindestens einem der Patentansprüche 1 bis 6, dadurch gekennzeichnet, dass man das Phosphoroxychlorid destillativ entfernt.

**8.** 3,5,6-Trichlor-picolinsäurechlorid als Verbindung der allgemeinen Formel I, worin $R_1$ und $R_3$ ein Chloratom und $R_2$ ein Wasserstoffatom bedeuten.

Europäisches Patentamt

# EUROPÄISCHER RECHERCHENBERICHT

Nummer der Anmeldung

EP 93 10 4574

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-A-2 713 316 (LONZA AG)<br>27. Oktober 1977<br>* Seite 2, Zeile 16 - Seite 2, Zeile 19 *<br>--- | 1-7 | C07D213/78<br>C07D241/24 |
| X,D | BERICHTE DER DEUTSCHEN CHEMISCHEN GESELLSCHAFT<br>Bd. 45, Nr. 314, 1912, WEINHEIM DE<br>Seiten 2456 - 2467<br>E. FISCHER ET AL. 'Verwandlung der Dihydrofuran-dicarbonsäure in Oxy-pyridin-carbonsäure'<br>* Seite 2461 *<br>--- | 1-7 | |
| A | GB-A-2 001 318 (SUMITOMO CHEMICAL COMPANY LIMITED)<br>31. Januar 1979<br>* Seite 1, Zeile 35 - Seite 1, Zeile 40 *<br>--- | | |
| A | US-A-4 081 451 (J. MAYER)<br>28. März 1978<br>* Beispiel 2 *<br>----- | 1-7 | |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 28 JUNI 1993 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)